# EUROPÄISCHE PATENTSCHRIFT

(11) **EP 1 383 485 B1**
(45) Veröffentlichungstag und Bekanntmachung des Hinweises auf die Patenterteilung: **09.09.2009**
(21) Anmeldenummer: 02730183.7
(22) Anmeldetag: 22.04.2002
(51) Int. Cl.: A61K 9/22, A61K 9/30, A61K 9/52

(54) **MAGENSAFTRESISTENTE VORRICHTUNG ZUR FREISETZUNG VON MUKOADHÄSIVEN WIRKSTOFFTRÄGERN UND VERFAHREN ZUR HERSTELLUNG DER MAGENSAFTRESISTENTEN VORRICHTUNG**
GASTRIC JUICE-RESISTANT DEVICE FOR RELEASING MUCOADHESIVE ACTIVE SUBSTANCE EXCIPIENTS AND METHOD FOR PRODUCING THIS GASTRIC JUICE-RESISTANT DEVICE
DISPOSITIF RESISTANT AU SUC GASTRIQUE SERVANT A LIBERER DES EXCIPIENTS DE PRINCIPES ACTIFS ADHERANT AUX MUQUEUSES, ET PROCEDE DE REALISATION DU DISPOSITIF RESISTANT AU SUC GASTRIQUE

(30) Priorität: 25.04.2001 DE 10120092
(43) Veröffentlichungstag der Anmeldung: 28.01.2004
(73) Patentinhaber: LTS LOHMANN Therapie-Systeme AG, 56626 Andernach (DE)
(72) Erfinder: SOLOMONIDOU, Despina, 79110 Freiburg (DE); KRUMME, Markus, 56567 Neuwied (DE); ASMUSSEN, Bodo, 56170 Bendorf-Sayn (DE); KREUTER, Jörg, 61350 Bad Homburg (DE)
(74) Vertreter: Flaccus, Rolf-Dieter
(86) Internationale Anmeldenummer: PCT/EP2002/004381
(87) Internationale Veröffentlichungsnummer: WO 2002/085333

(56) Entgegenhaltungen:
- EP-A- 0 516 141
- WO-A-00/32172
- WO-A-00/41740
- WO-A-00/66089
- WO-A-98/24412
- US-A- 4 289 751
- JUNGINGER H E ET AL: "BIOADHASIVE ARZNEISTOFFABGABESYSTEME UND ERZNEIFORMEN FUR PERORALE UND REKTALE ANWENDUNG" DEUTSCHE APOTHEKER ZEITUNG, DEUTSCHER APOTHEKER ZEITUNG, STUTTGART, DE, Bd. 130, Nr. 15, 12. April 1990 (1990-04-12), Seiten 791-801, XP000113829 ISSN: 0011-9857
- LEHR C M ET AL: "INTESTINAL TRANSIT OF BIOADHESIVE MICROSPHERES IN AN IN SITU LOOP IN THE RAT - A COMPARATIVE STUDY WITH COPOLYMERS AND BLENDS BASED ON POLY(ACRYLIC ACID)" JOURNAL OF CONTROLLED RELEASE, ELSEVIER SCIENCE PUBLISHERS B.V. AMSTERDAM, NL, Bd. 13, Nr. 1, 1. Juli 1990 (1990-07-01), Seiten 51-62, XP000142027 ISSN: 0168-3659

## Beschreibung

Die Erfindung betrifft eine magensaftresistente Vorrichtung zur Freisetzung von mukoadhäsiven Wirkstoffträgern, zur Erhöhung der Wirkstoffkonzentration im Darm, insbesondere im oberen Dünndarm. Desweiteren wird ein Verfahren zur Herstellung der erfindungsgemäßen Vorrichtung beschrieben.

Die lokale Behandlung des Darmes, z.B. der entzündeten oder infizierten Darmschleimhaut, erforderte bisher die häufige Gabe von relativ hohen Wirkstoffdosen. Bei herkömmlichen Arzneiformen haben die Wirkstoffe nur eine kurze lokale Einwirkdauer oder die Wirkstoffe gelangen erst nach systemischer Aufnahme über die Blutversorgung zum Wirkort. Mit der häufigen Gabe hoher Wirkstoffdosen ist ein hohes Risiko für die Patienten verbunden, durch unerwünschte Nebenwirkungen des hochdosierten Wirkstoffes beeinträchtigt zu werden.

Daneben sind Arzneistoffe bekannt, die in nennenswertem Ausmaß nur in einem sehr begrenzten Bereich des oberen Dünndarms resorbiert werden. Man spricht in einem solchen Fall von einem Resorptionsfenster. Ein Beispiel für eine solche Substanz, die nur in einem oberen Dünndarmabschnitt resorbiert wird, ist Riboflavin. Aber auch für die Wirkstoffe Captopril, Furosemid und Atenolol gibt es ein Resorptionsfenster. Derartige Arzneistoffe weisen eine geringe Bioverfügbarkeit auf, wenn sie mittels konventioneller Arzneiformen verabreicht werden.

Herkömmliche Arzneiformen sind daher für die Verabreichung solcher Substanzen oder zur Behandlung von Erkrankungen des oberen Dünndarms schlecht geeignet, da diese Darreichungsformen das Resorptionsfenster bzw. dem Wirkort bereits passieren, wenn ein großer Teil des Wirkstoffes noch nicht freigesetzt wurde, oder sie passieren das Resorptionsfenster so schnell, daß nur ein geringer Teil des Wirkstoffes vom Körper aufgenommen werden kann.

Aufgabe der vorliegenden Erfindung ist es, eine Arzneiform bereitzustellen, welche die Wirkstoffkonzentration am Wirk- bzw. Resorptionsort durch Verlängerung der Verweilzeit der Wirkstoffträger im Dünndarm, insbesondere im oberen Dünndarmabschnitt, erhöht, um dadurch die Bioverfügbarkeit des Wirkstoffes bzw. der Wirkstoffe zu verbessern, und welche die eingangs erwähnten Nachteile verhindert.

Die Aufgabe wird durch eine magensaftresistente Vorrichtung nach dem Anspruch 1 gelöst, wobei die Unteransprüche besonders nützliche Ausführungsformen der Erfindung betreffen.

Demnach umfaßt die erfindungsgemäße magensaftresistente Vorrichtung zumindest einen Wirkstoff in Form einer multipartikulären Zubereitung, deren Einzelpartikel mukoadhäsive Eigenschaften aufweisen.

Im Sinne der Erfindung umfaßt der Begriff "multipartikuläre Zubereitung" alle in der pharmazeutischen Technologie bekannten multipartikulären Arzneiformen. Dabei handelt es sich um Zubereitungsformen, bei denen jeweils eine Vielzahl von Einzelpartikeln eine Dosiseinheit des Wirkstoffes definiert. Zubereitungen dieser Art können beispielsweise Pellets, Mikrokapseln, Mikrosphären, Tabletten, Filme oder dergleichen sein.

Die Einzelpartikel der multipartikulären Zubereitung sind durch mukoadhäsive Eigenschaften gekennzeichnet. Die mukoadhäsiven Einzelpartikel der Vorrichtung dienen als die Wirkstoffträger und werden im Darmlumen freigesetzt. Nach Hydratisierung können die mukoadhäsiven Einzelpartikel am Darmepithel anhaften und den Wirkstoff kontrolliert vor Ort abgeben.

Somit kann durch die erfindungsgemäßen mukoadhäsiven Einzelpartikel die Verweilzeit des wirkstoffes am Wirkort oder in der Nähe des Wirkortes gegenüber konventionellen Arzneiformen wesentlich verlängert werden, so daß sehr viel geringere Dosen zum Erreichen des gleichen therapeutischen Erfolges notwendig sind. Damit verbunden wird auch das Risiko für die Patienten verringert, durch unerwünschte Nebenwirkungen hoch dosierter wirkstoffe beeinträchtigt zu werden.

Die Einzelpartikel der multipartikulären Wirkstoffzubereitung bestehen aus einem mindestens zweischichtigen Film. Dieser mindestens zweischichtige Film besteht aus mindestens einer wasserlöslichen, mukoadhäsiven Matrixschicht und einer hydrophoben, unlöslichen und wirkstoffundurchlässigen Rückschicht. Die mukoadhäsive Schicht ist durch ein hohes und langandauerndes Haftvermögen auf der Darmmukosa, ausgezeichnete Gewebeverträglichkeit und toxikologische Unbedenklichkeit gekennzeichnet. Ferner enthält die Matrixschicht einen oder mehrere Wirkstoffe. Die hydrophobe Rückschicht verhindert die rasche Auflösung und das Verkleben der mukoadhäsiven Schichten der Einzelpartikel miteinander. Die hydrophobe Rückschicht ist ebenfalls gut gewebeverträglich und toxikologisch unbedenklich. Zusätzlich bewirkt die Undurchlässigkeit der Rückschicht für den Wirkstoff, daß dessen Freisetzung nur in Richtung der Mukosa erfolgen kann. Dadurch gelangt der Wirkstoff gezielt an den Wirk- bzw. Resorptionsort und wird nicht unkontrolliert in das Darmlumen abgegeben. Durch Verwendung dieser mindestens zweischichtigen Filme wird die Bioverfügbarkeit von Wirkstoffen zur Behandlung des Darmes oder von Arzneistoffen mit einem Resorptionsfenster optimiert.

Die Größe der Einzelpartikel der multipartikulären Zubereitung kann vorzugsweise bis zu 8 mm Durchmesser betragen, besonders bevorzugt werden Partikelgrößen von bis zu 6 mm Durchmesser.

Der Einsatz von solch kleinen bzw. kleinflächigen Einzelpartikeln hat den Vorteil, daß die Darmperistaltik nur einen begrenzten Einfluß auf die Haftung der Einzelpartikel auf der Mukosa hat. Dadurch wird ein langanhaltender und enger Kontakt zwischen dem Darmepithel und den Einzelpartikeln gewährleistet, so daß ein sicherer Wirkstofftransfer von dem Einzelpartikel zum Wirk- bzw. Resorptionsort gewährleistet ist.

Im Gegensatz zu großflächigen Systemen können die erfindungsgemäßen kleinen bzw. kleinflächigen Einzelpartikel mit einem Durchmesser von nicht mehr als 8 mm auch nicht zu ausgedehnten Irritationen und Entzündungen der Schleimhaut führen. Die Verträglichkeit der erfindungsgemäßen Vorrichtung ist gegenüber herkömmliche Arzneiformen also erheblich verbessert.

Bevorzugt weist die erfindungsgemäße Vorrichtung eine magensaftresistente Hülle auf, die aus einem Polymermaterial besteht und die Wirkstoffträger platzsparend umgibt. Die Polymerhülle besteht aus einem Material, das im sauren Magenmilieu resistent ist, jedoch im Darmsaft rasch zerfällt und dabei die mukoadhäsiven Wirkstoffträger vollständig in das Darmlumen freisetzt. Dazu ist es für die Funktion der Polymerhülle unabdingbar, daß sie im Magensaft bei Körpertemperaturen, also bei Temperaturen von bis zu etwa 40°C, unlöslich und impermeabel ist. Als Polymermaterialien, die für die magensaftresistente Hülle verwendet werden können, kommen beispielsweise Methacrylsäure/Ethylacrylat-Copolymere, Hydroxypropyl-methylcellulose-phthalat, Celluloseacetat-phthalat oder Methacrylsäureester in Betracht.

Mit einer derartigen Polymerhülle versehen können auch solche Wirkstoffe appliziert werden, die im sauren Magenmilieu eine vergleichsweise geringe Stabilität aufweisen oder durch den Einfluß von im Magenmilieu auftretenden Enzymen einen Abbau erfahren, die allerdings von der Darmschleimhaut besonders gut aufgenommen werden. Beispiele für derartige Substanzen sind Proteine, Peptide oder Hormone, wie beispielsweise Insulin oder Octreotid.

Zur Einstellung der notwendigen mechanischen und physikochemischen Eigenschaften der Hülle, wie Festigkeit, Flexibilität, Siegelfähigkeit, Hydrophilie, usw., kann diese neben dem Polymermaterial auch noch ein weiteres modifizierendes Polymer oder weitere übliche Hilfsstoffe enthalten. Beispiele für Hilfsstoffe, die erforderlich sein können, sind weichmacher, Netzmittel, Mattierungsmittel, Farbstoffe, Stabilisatoren und Geschmackskorrigenzien.

Eine weitere erfindungsgemäße Variante der Polymerhülle weist einen mehrschichtigen Aufbau auf. Ein solcher Aufbau kann dadurch bedingt sein, daß es aus fertigungstechnischen Gründen vorteilhaft oder sogar notwendig ist, die Polymerhülle aus mehreren Schichten aufzubauen.

Damit die erfindungsgemäße Vorrichtung von Patienten besser und angenehmer geschluckt werden kann, ist es zweckmäßig, daß die Polymerhülle so geformt ist, daß eine einfache und sichere perorale Applikation ermöglicht wird. Hierfür wird erfindungsgemäß die Darreichungsform einer Kapsel bevorzugt, deren Herstellung nach standardisierten Verfahren erfolgt und als Darreichungsform von Patienten weitgehend sehr gut akzeptiert ist.

Die Vorrichtung enthält ein Treibmittel, das die Dispergierung der multipartikulären wirkstoffhaltigen Zubereitung bewirkt. Zusätzlich beschleunigt das Treibmittel den Zerfall der Polymerhülle und unterstützt die Freisetzung der Einzelpartikel. Durch Zutritt von Flüssigkeit wird das Expansionsmittel aktiviert.

Bei dem Treibmittel handelt es sich um eine bei Kontakt mit Körperflüssigkeiten (z.B. Darmsaft) gaserzeugende Komponente. Als Expansionsmechanismus der expandierbaren Komponente der Vorrichtung dient die Gaserzeugung bei Kontakt mit Darmsaft.

Die einzelnen Wirkstoffträger erhalten durch das entstehende Gas einen Impuls zum Austreten aus der Polymerumhüllung, so daß sie schnell und vollständig aus der Polymerumhüllung austreten. Dadurch wird gleichzeitig die Gefahr verringert, daß sich die wirkstoffhaltigen Zubereitungen durch gegenseitige Berührung miteinander verkleben.

Während sich aus physiologischer Sicht verschiedene Gase eignen, darunter z.B. Stickstoff, wird die Erzeugung von Kohlendioxid besonders bevorzugt, da sich dieses Gas leicht aus unbedenklichen Treibmitteln gewinnen läßt. Als Substanzen, aus denen Kohlendioxid freigesetzt werden kann, eignen sich verschiedene Carbonate, wie Natriumcarbonat, Kaliumcarbonat oder Ammoniumcarbonat, sowie entsprechende Hydrogencarbonate. Der guten Verträglichkeit und der hohen Ausbeute halber wird jedoch erfindungsgemäß ein Hydrogencarbonat, z.B. Natriumhydrogencarbonat, bevorzugt.

Bei der Zusammensetzung des Treibmittels sind außerdem die physiologischen Bedingungen im oberen Dünndarm zu berücksichtigen. Der pH-Wert im Duodenum liegt im nüchternen Zustand zwischen 5,5, und 6,5, während in der postprandialen Phase ein Abfall unter pH 5,4 beobachtet wird. Im Jejunum werden pH-Werte zwischen 6,3 und 7,3 gemessen. Aus diesen Gründen ist der Zusatz einer Säurekomponente in das Treibmittel erforderlich, die bei Zutritt von Wasser die Gaserzeugung bewirkt. Als Säurekomponenten können beispielsweise Zitronensäure, Weinsäure oder Natriumdihydrogenphosphat eingesetzt werden.

Auch das erfindungsgemäße Treibmittel liegt als multipartikuläre Zubereitung vor. Es kann notwendig sein, einen Teil des Treibmittels schnell zur Wirkung zu bringen, um z.B. den Zerfall der Polymerumhüllung zu beschleunigen. Darüberhinaus kann das Treibmittel als Pulvermischung oder als eine unter Verwendung von üblichen pharmazeutischen Hilfsstoffen geformte Zubereitung, z. B. Preßlinge oder Pellets, vorliegen. Außerdem ist es im Sinne dieser Erfindung möglich, das Treibmittel in der multipartikulären wirkstoffhaltigen Zubereitung einzuarbeiten. Besonders vorteilhaft ist jedoch die räumliche Trennung von Wirkstoff und Expansionsmitteln in der Zubereitung, denn sie verringert die Gefahr von Inkompatibilitäten.

Für ein einwandfreies Freisetzen der Einzelpartikel aus der erfindungsgemäßen Vorrichtung ist die räumliche Anordnung der wirkstoffhaltigen sowie der treibmittelhaltigen Zubereitungen von großer Bedeutung, da die Wirkstoffträger mit der mukoadhäsiven Schicht voran aus der Kapsel herausgeschleudert werden sollen. Es liegt eine alternierend geschichtete Anordnung der wirkstoffhaltigen und der treibmittelhaltigen Zubereitungen innerhalb der Vorrichtung vor, so daß die einzelnen wirkstoffhaltigen Zubereitungen nacheinander mit der mukoadhäsiven Schicht voran aus der Vorrichtung freigesetzt werden. Diese Ausführungsform verhindert zudem, daß die einzelnen wirkstoffhaltigen Zubereitungen nach ihrer Freisetzung mit ihren wirkstoffhaltigen Schichten aneinanderhaften.

Die erfindungsgemäße Vorrichtung bietet die Möglichkeit, die Freisetzungsgeschwindigkeit des Wirkstoffes bzw. der Wirkstoffe durch die Art und Zusammensetzung der multipartikulären Zubereitung (Wirkstoffträger) zu steuern, unabhängig von den Eigenschaften der Polymerhülle. Die Polymerhülle kann also hinsichtlich anderer wichtiger Aspekte wie Festigkeit, Flexibilität, Siegelfähigkeit oder Magensaftresistenz optimiert werden und muß nicht auf die Kontrolle der Freisetzungsgeschwindigkeit eingestellt werden.
Die von der Polymerumhüllung weitgehend unabhängige Steuerungsmöglichkeit der Freisetzungsgeschwindigkeit ist insbesondere deshalb von Bedeutung, weil an dieselbe Umhüllung die Forderung gestellt wird, daß sie erst bei Kontakt mit Darmsaft Wasser aufnimmt bzw. die Diffusion von Wasser ermöglicht, im Magensaft jedoch unlöslich und impermeabel ist.

Erfindungsgemäße Vorrichtungen können zu verschiedenen Zwekken therapeutisch eingesetzt werden. Wichtige Einsatzgebiete sind die Applikation von Wirkstoffen zur lokalen Behandlung des Darmes, insbesondere des oberen Dünndarmabschnittes, bei Infektionen der Darmschleimhaut sowie bei entzündlichen und chronisch-entzündlichen Darmerkrankungen, wie beispielsweise bei Colitis ulcerosa oder Enteris regionalis. Dabei führt die erfindungsgemäße Vorrichtung zu einer Verbesserung der Bioverfügbarkeit von Wirkstoffen mit einem sog. Resorptionsfenster im oberen Dünndarmabschnitt. Außerdem ist sie für die Applikation von Wirkstoffen prädestiniert, die im Magenmilieu labil sind und im oberen Dünndarmabschnitt gut resorbiert werden.

Die Zeichnungen veranschaulichen eine besonders gut geeignete Ausführungsform der erfindungsgemäßen Vorrichtung. Es zeigt
- Figur 1: einen schematischen Schnitt durch eine erfindungsgemäße Vorrichtung,
- Figur 2: zwei unterschiedliche Ausführungsformen der wirkstoffhaltigen Einzelpartikel im Zustand der Wirkstoffabgabe.

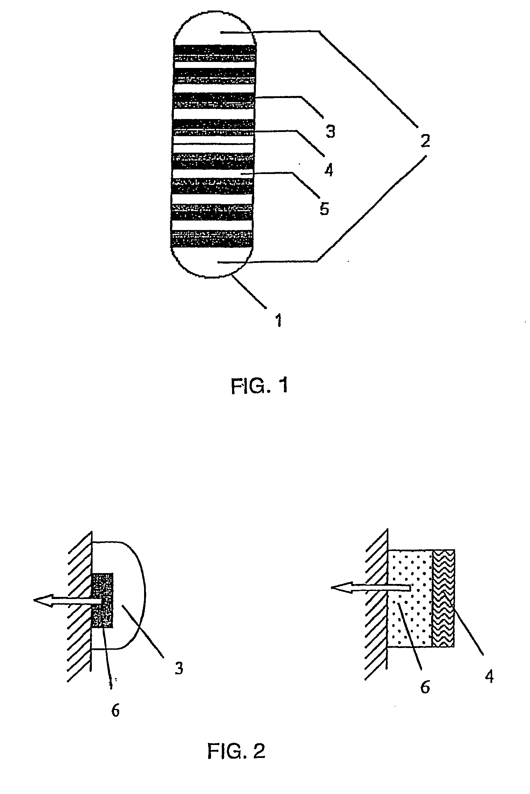

Bei der vorliegenden Erfindung, die beispielhaft in Figur 1 dargestellt ist, befindet sich innerhalb einer magensaftresistenten, jedoch für Darmsaft permeablen Polymerhülle (1) ein Vorrat an gaserzeugenden Komponenten (2 und 5) sowie eine Anzahl von filmartigen, geschichteten Einzelpartikeln, welche aus einer mukoadhäsiven, wirkstoffhaltigen Schicht (3) und einer die Richtung der Wirkstofffreisetzung steuerenden Rückschicht (4) bestehen. Dabei kann der Wirkstoff (6) in der filmartigen Komponente eingebettet vorliegen, wie in Figur 2 dargestellt ist.

Die erfindungsgemäße Vorrichtung kann durch ein Verfahren hergestellt werden, in dem die folgenden Arbeitsschritte aufeinander folgen:

Die erfindungsgemäße Vorrichtung kann durch ein Verfahren hergestellt werden, in dem die folgenden Arbeitsschritte aufeinander folgen:
a. Transfer eines bahnförmigen Polymermaterials auf eine mit Bohrungen versehene Formplatte und Anlegen eines Vakuums, um die Kompartimente der Polymerhülle zu formen,
b. alternierendes Einfüllen von wirkstoffhaltiger Zubereitung und treibmittelhaltiger Zubereitung,
c. Darüberlegen einer zweiten Polymerbahn und Verschließen der Kompartimente durch Siegelung unter Anwendung von Wärme und Druck, und
d. Separieren der einzelnen Vorrichtungen durch Stanzen oder Schneiden.

Nachfolgend wird die erfindungsgemäße magensaftresistente Vorrichtung zur Freisetzung von Wirkstoffen mit verzögerter Darmpassage anhand von Ausführungsbeispielen zur Herstellung mit mukoadhäsiven Wirkstoffzubereitungen anschaulich beschrieben. Dabei sind die nachfolgenden Beispiele nicht als die vorliegende Erfindung einschränkend aufzufassen.

### 1. Herstellung und Charakterisierung von zweischichtigen mukoadhäsiven Wirkstoffträgern

Als matrixbildende Polymere der mukoadhäsiven Schicht der Filme wurden Polyvinylpyrrolidon (PVP) bzw. Polyvinylalkohol (PVA) eingesetzt. Carbopol^{®} 934P, Carbopol^{®} 974NF und Polycarbophil (Noveon^{™} AA1), sowie auch Natriumcarboxymethylcellulose (CMC) dienten als mukoadhäsive Polymere. Polyethylenglykol (PEG 600) wurde als Weichmacher verwendet. Die Konzentration des mukoadhäsiven Polymers wurde zwischen 0 und 20 Gew.-% variiert. Zur Herstellung der lipophilen, nicht mukoadhäsiven Schicht wurden Celluloseacetatbutyrat (CAB) als Filmbildner, Titandioxid als Pigment und Diethylphthalat als Weichmacher eingesetzt.

Das Polymer wurde zunächst unter starkem Rühren auf das Lösungsmittel aufgestreut. Als Lösungsmittel für Polyvinylalkohol, Polyvinylpyrrolidon und Natriumcarboxymethylcellulose diente destilliertes Wasser; für Celluloseacetatbutyrat wurde ein Gemisch aus Ethylacetat und absolutem Alkohol eingesetzt. Im Fall der CAB-Lösung wurde nach dem Polymer auch das Pigment und der Weichmacher hinzugefügt und bis zur homogenen Verteilung und Auflösung weitergerührt. Zur vollständigen Auflösung der Polymere wurde etwa 3 - 4 h bei Raumtemperatur weitergerührt. Im Falle von PVA und PVP wurden die Lösungen während dieser Zeit im Wasserbad auf 60 °C erwärmt.
Zur Herstellung der Polyacrylsäure-Suspensionen wurden die Polymerpulver (1 Gew.-%) in destilliertem Wasser dispergiert und anschließend unter starkem Rühren mit 1 N NaOH-Lösung wurde der pH-Wert der Dispersion auf 4,8, 5,5, 6,8 und 7,5 eingestellt.
Wirkstoffhaltige Polymerzubereitungen wurden durch Zugabe von Riboflavin in die wäßrige Polyvinylpyrrolidon- bzw. Polyvinylalkohol-Lösung und durch Rühren unter Lichtabschluß bis zur Auflösung des Wirkstoffs hergestellt.
Die Beschichtungsmasse zur Herstellung der Filme wurde aus entsprechenden Mengen an Lösungen des mukoadhäsiven Polymers, des Filmbildners und des Weichmachers durch Mischen in einem Laborreaktor mit eingebautem Rührwerk bis zur Homogenität hergestellt.
Die mukoadhäsiven Filme wurden durch Beschichten einer Trägerfolie hergestellt. Dazu wurde die Trägerfolie (Hostapan^{®} RN 100) auf eine horizontal ausgerichtete Glasplatte aufgelegt und die Masse für die mukoadhäsive Schicht mit Hilfe einer Ziehrakel bei konstanter Geschwindigkeit aufgebracht. Die wirkstofffreien Folien wurden im Trockenschrank bei 60 °C über 25 min, die Riboflavin-haltigen Folien an der Luft unter Lichtabschluß über Nacht getrocknet. Die getrockneten Folien wurden anschließend in gleicher Weise mit der Masse für die lipophile Schicht beschichtet. Nach der Trocknung im Trockenschrank bei 60 °C über 15 min wurden die Filme mit einem Henkellocheisen in der gewünschten Form aus dem Laminat ausgestanzt.
Die Charakterisierung der Wirkstoffträger erfolgte im Hinblick auf die Untersuchung der Adhäsionskraft und Adhäsionsdauer auf Duodenalmukosa, Bestimmung der Wasseraufnahmekapazität der mukoadhäsiven Schicht sowie auch auf die Untersuchung der Wirkstofffreisetzung und Wirkstoffpermeation. Die Untersuchungen haben gezeigt, daß Formulierungen auf PVP-Basis erheblich bessere mukoadhäsive Eigenschaften aufweisen als solche mit PVA als Filmmatrix. Darüberhinaus konnte gezeigt werden, daß ein Zusammenhang zwischen der Konzentration des mukoadhäsiven Polymers und den mukoadhäsiven Eigenschaften der Formulierung (Adhäsionskraft und Adhäsionsdauer) besteht: die Konzentration hat generell einen positiven Einfluß auf das Adhäsionspotential. Der Neutralisationsgrad der Polyacrylsäure-Derivate hat ebenfalls einen Effekt auf die mukoadhäsiven Eigenschaften der filmartigen Formulierungen mit einem Optimum bei pH 5,5 und 6,8 der wäßrigen Polyacrylsäure-Disperion. Weiterhin haben wir erwiesen, daß die Freisetzung des Wirkstoffes rasch und vollständig erfolgt.

### 2. Herstellung von treibmittelhaltigen Tabletten

Zur Herstellung von treibmittelhaltigen Tabletten wurden Maisstärke als Füllstoff, Polyethylenglykol (PEG 400) als Gleitmittel, und ein Gemisch aus Natriumhydrogencarbonat, kristalliner Weinsäure und Natriumdihydrogenphosphat als Treibmittel-Mischung eingesetzt.
Biplane Tabletten mit 8 mm Durchmesser wurden durch Direkttablettierung mit einer instrumentierten Exzentertablettenpresse hergestellt. Die pulverförmigen Hilfsstoffe (Bindemittel, Gleitmittel und Säurekomponente) wurden im Taumelmischer 8 min lang vermischt. Anschließend wurde Natriumhydrogencarbonat dazugegeben und erneut gemischt. Die Tabletten wurden im Exsikkator über Blaugel gelagert.

### 3. Herstellung und Prüfung von magensaftresistenten Kapseln

Zur Herstellung der magensaftresistenten Kapseln als primäre Arzneiform zur Applikation der mukoadhäsiven Filme wurde die wäßrige Dispersion eines Copolymers aus Methacrylsäure und Ethylacrylat (Kollicoat^{®} MAE) eingesetzt. Zur Erhöhung der Viskosität wurde Natriumcarboxymethylcellulose (CMC) eingesetzt. Als Weichmacher wurde Diethylphthalat benutzt. Magensaftresistente Kapseln wurden aus Kollicoat^{®} MEA, CMC und Diethylphthalat im Verhältnis 60:10:30 (Massenanteile) hergestellt. Die wäßrige CMC-Lösung wird vorgelegt und die 30 Gew.-% Kollicoat^{®} MAE-Dispersion sowie der Weichmacher unter Rühren dazugegeben. Die homogene Dispersion wird mit Hilfe einer Ziehrakel bei konstanter Geschwindigkeit auf eine Trägerfolie aufgetragen. Die Folien wurden anschließend im Trokkenschrank bei 60 °C 30 min lang getrocknet.
Die Folien wurden auf die Füllapparatur transferiert. Bei dieser Apparatur handelt es sich um eine Formplatte aus Metall mit kapselförmigen, ovalen Bohrungen. Durch Anlegen von Vakuum in den einzelnen Bohrungen wurden in der Folie Mulden geformt, die daraufhin alternierend mit den treibmittelhaltigen Tabletten und den zweischichtigen, wirkstoffhaltigen Filmen gefüllt wurden. An beiden Enden der Kapsel befindet sich zusätzlich Treibmittelmischung. Anschließend wurde eine zweite Folie aufgelegt, mit der die Kapseln durch Erwärmen und Aufpressen einer passenden Deckplatte verschlossen und ausgestanzt wurden.
Diese spezielle Anordnung dient zum einen der raschen Freisetzung und zum anderen zur Dispergierung der Filme beim Zerfall der Kapselwand. Durch den Zutritt von Flüssigkeit wird der Treibmittelsatz der Tabletten und der Pulvermischung aktiviert. Die einzelnen Filme erhalten durch das entstehende CO₂ einen Impuls zum Austreten aus der Kapsel. Der Zerfall der Kapsel beginnt an beiden Enden, während der Kapselmantel zunächst bestehen bleibt. Die Filme werden mit der mukoadhäsiven Schicht voran aus der Kapsel herausgeschleudert. Dabei wird gleichzeitig die Gefahr verringert, daß sich die Filme durch gegenseitige Berührung miteinander verkleben. Ein Vorteil dieser Applikationsform besteht darin, daß der Zerfall der Kapsel keinen limitierenden Faktor für die Wirkstofffreisetzung darstellt: Die Kapsel ist in der Lage, die Filme schnell, einzeln und vollständig freizusetzen. Dies ergaben ex-vivo Versuche mit Schweinedarm.
Die Kapseln wurden zusätzlich (in Anlehnung an das Europäische Arzneibuch) in einer Zerfallsapparatur zunächst in 0,1 N HCl bei 37 °C 2 h lang geprüft. Dabei hat keine Kapsel Anzeichen von Zerfall und Wasserdurchlässigkeit aufgewiesen. Anschließend wurde mit Phosphat-Pufferlösung, pH 6,8, 60 min lang geprüft. Die Kapseln zerfielen innerhalb kurzer Zeit, und entsprachen somit den Anforderungen des Arzneibuches.

## Patentansprüche

1. Magensaftresistente Vorrichtung zur Freisetzung von Wirkstoffträgern mit verzögerter Darmpassage aus einer magensaftresistenten Hülle, **dadurch gekennzeichnet, daß** der mindestens einen Wirkstoff enthaltende Wirkstoffträger in Form einer multipartikulären Zubereitung vorliegt, deren Einzelpartikel aus einem mindestens zweischichtigen Film bestehen, von denen mindestens eine Schicht mukoadhäsive Eigenschaften aufweist, und die Vorrichtung ein bei Kontakt mit Flüssigkeit gaserzeugendes Treibmittel enthält, das als weitere multipartikuläre Zubereitung in der Vorrichtung enthalten ist und die Dispergierung der multipartikulären wirkstoffhaltigen Zubereitung bewirkt, wobei die einzelnen wirkstoffhaltigen Zubereitungen und die einzelnen treibmittelhaltigen Zubereitungen alternierend in der Vorrichtung vorliegen.

2. Vorrichtung nach Anspruch 1, **dadurch gekennzeichnet, daß** die Größe der Einzelpartikel der multipartikulären Zubereitung bis zu 8 mm Durchmesser beträgt, vorzugsweise bis zu 6 mm Durchmesser.

3. Vorrichtung nach Anspruch 1 oder 2, **dadurch gekennzeichnet, daß** die Einzelpartikel der multipartikulären Zubereitung den Wirkstoff zumindest in einem Teilbereich eingebettet enthalten.

4. Vorrichtung nach einem der vorhergehenden Ansprüche, **dadurch gekennzeichnet, daß** die magensaftresistente Hülle aus einem Polymermaterial besteht.

5. Vorrichtung nach Anspruch 4, **dadurch gekennzeichnet, daß** die Polymerhülle ein- oder mehrschichtig aufgebaut ist.

6. Vorrichtung nach Anspruch 4 oder 5, **dadurch gekennzeichnet, daß** die Polymerhülle aus einem hydrophilen, im Magensaft und bei Temperaturen bis 40°C resistenten, unlöslichen und impermeablen, jedoch im Darmsaft löslichen und/oder permeablen Polymer besteht.

7. Vorrichtung nach einem der Ansprüche 4 bis 6, **dadurch gekennzeichnet, daß** die Polymerhülle so geformt ist, daß eine perorale Applikation ermöglicht wird.

8. Vorrichtung nach Anspruch 1, **dadurch gekennzeichnet, daß** das Treibmittel vorzugsweise Hydrogencarbonat enthält und/oder bei Kontakt mit Darmsaft Kohlendioxid erzeugt.

9. Vorrichtung nach einem der vorangehenden Ansprüche, **dadurch gekennzeichnet, daß** das Treibmittel zusätzlich eine Säurekomponente enthält.

10. Vorrichtung nach einem der vorangehenden Ansprüche, **dadurch gekennzeichnet, daß** die bei Kontakt mit Darmsaft gaserzeugende Komponente in den Wirkstoffträgern eingebettet vorliegt.

11. Vorrichtung nach einem der vorangehenden Ansprüche, **dadurch gekennzeichnet, daß** sie mindestens einen Wirkstoff enthält, der wirksam gegen Darmerkrankungen des oberen Dünndarmabschnittes eingesetzt werden kann.

12. Vorrichtung nach einem der vorhergehenden Ansprüche, **dadurch gekennzeichnet, daß** sie mindestens einen Wirkstoff enthält, der im oberen Dünndarmabschnitt schneller und/oder in höherem Ausmaß als in den übrigen Abschnitten des Darmes resorbiert wird.

13. Vorrichtung nach einem der vorhergehenden Ansprüche, **dadurch gekennzeichnet, daß** sie mindestens einen Wirkstoff enthält, der im Magen eine geringe Stabilität aufweist oder der durch den Einfluß von im Magen auftretenden Substanzen ein herabgesetztes Ausmaß an Bioverfügbarkeit erfährt und der im oberen Dünndarmabschnitt gut resorbiert wird.

14. Verfahren zur Herstellung einer magensaftresistenten Vorrichtung, die aus mindestens einem Wirkstoff in Form einer multiparikulären Zubereitung, deren Einzelpartikel mukoadhäsive Eigenschaften aufweisen, und einem bei Kontakt mit Flüssigkeit gaserzeugenden Treibmittel besteht, welche von einer magensaftresistenten, darmsaftlöslichen Polymerhülle umgeben sind, das die folgenden Schritte umfaßt:
a. Transfer eines bahnförmigen Polymermaterials auf eine mit Bohrungen versehene Formplatte und Anlegen eines Vakuums, um die Kompartimente der Polymerhülle zu formen,
b. alternierendes Einfüllen von wirkstoffhaltiger Zubereitung und treibmittelhaltiger Zubereitung,
c. Darüberlegen einer zweiten Polymerbahn und Verschließen der Kompartimente durch Siegelung unter Anwendung von Wärme und Druck, und
d. Separieren der einzelnen Vorrichtungen durch Stanzen oder Schneiden.

## Claims

1. Gastric juice-resistant device for releasing active substance excipients which have a delayed intestinal passage from a gastric juice-resistant enclosure, **characterized in that** the active substance excipient containing at least one active substance is present in the form of a multiparticulate preparation whose individual particles consist of a film of at least two layers, of which at least one has mucoadhesive properties, and **in that** said device contains a blowing agent which generates gas on contact with a liquid, said blowing agent being contained in said device as a further multiparticulate preparation and causing the dispersion of the multiparticulate active substance-containing preparation, and the individual active substance-containing preparations and the individual blowing agent-containing preparations being alternately present in said device.

2. Device according to claim 1, **characterized in that** the size of the individual particles of the multiparticulate preparation is up to 8 mm in diameter, preferably up to 6 mm in diameter.

3. Device according to claim 1 or 2, **characterized in that** the individual particles of the multiparticulate preparation contain the active substance embedded in at least a partial region thereof.

4. Device according to any one of the preceding claims, **characterized in that** the gastric juice-resistant enclosure consists of a polymer material.

5. Device according to claim 4, **characterized in that** the polymer enclosure has a single-layered or multi-layered structure.

6. Device according to claim 4 or 5, **characterized in that** the polymer enclosure consists of a hydrophile, insoluble and impermeable polymer which is resistant in gastric juice and at temperatures of up to 40 °C, but is soluble and/or permeable in intestinal juice.

7. Device according to any one of claims 4 to 6, **characterized in that** the polymer enclosure is shaped such that a peroral application is made possible.

8. Device according to claim 1, **characterized in that** the blowing agent preferably contains hydrogen carbonate, and/or generates carbon dioxide on contact with intestinal juice.

9. Device according to any one of the preceding claims, **characterized in that** the blowing agent additionally contains an acid component.

10. Device according to any one of the preceding claims, **characterized in that** the component which on contact with intestinal juice produces a gas is embedded in the active substance excipients.

11. Device according to any one of the preceding claims, **characterized in that** it contains at least one active substance which can be effectively used for treating intestinal affections of the upper section of the small intestine.

12. Device according to any one of the preceding claims, **characterized in that** it contains at least one active substance which is absorbed more rapidly and/or to a greater extent in the upper section of the small intestine than in the other sections of the intestine.

13. Device according to any one of the preceding claims, **characterized in that** it contains at least one active substance which has low stability in the stomach or which due to the influence of substances occurring in the stomach experiences a reduced extent of bioavailability and which is absorbed well in the upper section of the small intestine.

14. Process for the production of a gastric juice-resistant device, which device consists of at least one active substance in the form of a multiparticulate preparation whose individual particles have mucoadhesive properties, and of a blowing agent which on contact with a liquid produces a gas, said individual particles being enclosed by a gastric juice-resistant, intestinal juice-soluble polymer enclosure, said process comprising the following steps:
a. transfer of a polymer material in web form to a moulding board provided with bores, and applying a vacuum to form the compartments of the polymer enclosure;
b. alternately filling-in the active substance-containing preparation and the blowing agent-containing preparation;
c. superposing a second polymer web, and closing the compartments by sealing with application of heat and pressure; and
d. separating the individual devices by punching or cutting.

## Revendications

1. Dispositif résistant au suc gastrique pour la libération de supports de substances actives, dont le transit est retardé, constitué d'une enveloppe résistant au suc gastrique, **caractérisé en ce que** le support de substances actives contenant au moins une substance active est présent sous la forme d'une préparation multiparticulaire, dont les particules individuelles sont constituées d'au moins un film bicouche dont au moins une couche présente des propriétés mucoadhésives, et le dispositif contient un agent moussant générant un gaz par contact avec un liquide, qui est contenu dans le dispositif à titre de préparation multiparticulaire supplémentaire et qui déclenche la mise en dispersion de la préparation multiparticulaire contenant une ou plusieurs substances actives, les préparations individuelles contenant une ou plusieurs substances actives et les préparations individuelles contenant un ou plusieurs agents moussants étant présentes en alternance dans le dispositif.

2. Dispositif selon la revendication 1, **caractérisé en ce que** la dimension des particules individuelles de la préparation multiparticulaire s'élève, en ce qui concerne le diamètre, jusqu'à 8 mm, de préférence jusqu'à 6 mm.

3. Dispositif selon la revendication 1 ou 2, **caractérisé en ce que** les particules individuelles de la préparation multiparticulaire contiennent la substance active à l'état enrobé au moins dans une zone partielle.

4. Dispositif selon l'une quelconque des revendications précédentes, **caractérisé en ce que** l'enveloppe résistant au suc gastrique est constituée d'une matière polymère.

5. Dispositif selon la revendication 4, **caractérisé en ce que** l'enveloppe polymère est composée d'une seule couche ou de plusieurs couches.

6. Dispositif selon la revendication 4 ou 5, **caractérisé en ce que** l'enveloppe polymère est constituée d'un polymère hydrophile, insoluble et imperméable qui résiste dans le suc gastrique et jusqu'à des températures de 40 °C, mais qui est néanmoins soluble et/ou perméable dans le suc intestinal.

7. Dispositif selon l'une quelconque des revendications 4 à 6, **caractérisé en ce que** l'enveloppe polymère est configurée de telle sorte qu'une application par voie orale est possible.

8. Dispositif selon la revendication 1, **caractérisé en ce que** l'agent moussant contient de préférence un hydrogénocarbonate e/ou génère du dioxyde de carbone lors d'un contact avec le suc intestinal.

9. Dispositif selon l'une quelconque des revendications précédentes, **caractérisé en ce que** l'agent moussant contient en outre un composant acide.

10. Dispositif selon l'une quelconque des revendications précédentes, **caractérisé en ce que** le composant générant un gaz lors d'un contact avec le suc intestinal est présent à l'état enrobé dans les supports de substances actives.

11. Dispositif selon l'une quelconque des revendications précédentes, **caractérisé en ce qu'**il contient au moins une substance active qui peut être mise en oeuvre pour agir contre des affections intestinales du tronçon supérieur de l'intestin grêle.

12. Dispositif selon l'une quelconque des revendications précédentes, **caractérisé en ce qu'**il contient au moins une substance active qui est résorbée, dans le tronçon supérieur de l'intestin grêle, plus rapidement que dans autres tronçons de l'intestin et/ou dans une mesure supérieure à celle des autres tronçons de l'intestin.

13. Dispositif selon l'une quelconque des revendications précédentes, **caractérisé en ce qu'**il contient au moins une substance active qui présente, dans l'estomac, une faible stabilité, ou qui subit, par l'influence de substances présentes dans l'estomac, une diminution de l'ampleur de sa biodisponibilité et qui est bien résorbée dans le tronçon supérieur de l'intestin grêle.

14. Procédé pour la fabrication d'un dispositif résistant au suc gastrique, qui est constitué par au moins une substance active sous la forme d'une préparation multiparticulaire, dont les particules individuelles présentent des propriétés mucoadhésives, et par un agent moussant générant un gaz lors d'un contact avec un liquide, qui sont entourés par une enveloppe polymère soluble en présence de suc intestinal et résistant au suc gastrique, qui comprend les étapes suivantes :
a. le transfert d'une matière polymère en forme de bande sur une plaque de façonnement munie d'alésages et l'application d'un vide pour façonner les compartiments de l'enveloppe polymère;
b. le transvasement en alternance de la préparation contenant une ou plusieurs substances actives et de la préparation contenant un ou plusieurs agents moussants ;
c. la superposition d'une deuxième bande polymère et la fermeture des compartiments par scellement en utilisant de la chaleur et de la pression ; et
d. la séparation des dispositifs individuels par emboutissage ou par découpe.
